Europäisches Patentamt

European Patent Office

Office européen des brevets

(11) Veröffentlichungsnummer: **0 261 318 B1**

# (12) EUROPÄISCHE PATENTSCHRIFT

(45) Veröffentlichungstag der Patentschrift: **22.05.91**

(51) Int. Cl.⁵: **A61M  5/32**

(21) Anmeldenummer: **87108997.5**

(22) Anmeldetag: **23.06.87**

(54) **Selbsttätige Injektionsvorrichtung und -Patrone mit Nadel.**

(30) Priorität: **01.07.86 DE 3622399**

(43) Veröffentlichungstag der Anmeldung:
**30.03.88 Patentblatt  88/13**

(45) Bekanntmachung des Hinweises auf die
Patenterteilung:
**22.05.91 Patentblatt  91/21**

(84) Benannte Vertragsstaaten:
**AT BE CH DE ES FR GB GR IT LI LU NL SE**

(56) Entgegenhaltungen:
**EP-A- 0 144 625
DE-A- 1 491 842
DE-A- 1 934 117
FR-A- 2 237 643
FR-A- 2 239 258**

(73) Patentinhaber: **Schlüter, Eberhardt, Dipl.-Kfm.
Elfenbeinweg 13
W-2000 Hamburg 65(DE)**

(72) Erfinder: **Schlüter, Eberhardt, Dipl.-Kfm.
Elfenbeinweg 13
W-2000 Hamburg 65(DE)**
Erfinder: **Scheller, Albert, Dr.-Dipl.-Chem.
Hauptstrasse 93
W-7560 Gaggenau(DE)**
Erfinder: **Sprenger, Rolf
Im Rehgarten 7
W-7560 Gaggenau/Winkel(DE)**

(74) Vertreter: **Patentanwälte Wenzel & Kalkoff
Grubes Allee 26 Postfach 730466
W-2000 Hamburg 73(DE)**

## Beschreibung

Die Erfindung betrifft eine selbsttätige Injektionsvorrichtung mit einer Patrone oder Ampulle, die in einem Gehäuse gelagert ist, Injektionsstoff aufnimmt, ein Hubelement aufweist, eine einen langgestreckten Nadelkörper und eine Kanüle umfassende Injektionsnadel, die durch das Hubelement bewegbar ist und im Bereich ihres antriebsseitigen Endes die Kanülen-Eintrittsöffnung für den Injektionsstoff aufweist, umschließt und eine an der Seite der Nadelspitze angeordnete sowie den Nadelschaft durchlassende Dichtung umfaßt, mit einer an dem Gehäuse gelagerten, das Hubelement mit einer Kraft beaufschlagenden Injektions-Druckeinrichtung, mit einer die Druckeinrichtung in Betrieb setzenden Auslöseeinrichtung und mit einer Sicherheitseinrichtung zum Blockieren der Auslöseeinrichtung sowie eine Patrone oder Ampulle zur Verwendung in Injektionsvorrichtungen, insbesondere in einer selbsttätigen Injektionsspritze, wobei ein in der Patrone oder Ampulle befindlicher Injektionsstoff durch eine Kanüle einer an der Patrone oder Ampulle gelagerten Injektionsnadel infolge der Hubbewegung eines Hubelementes wie insbesondere eines Stopfens oder Bolzens austreibbar ist.

Eine automatische Selbstinjektionsspritze eröffnet Laien die Möglichkeit einer subkutanen oder auch intramuskulären Eigeninjektion. Besondere Bedeutung besteht in der Injektion von Atropinsulfat als Nervengasprevention. Zudem wird Atropin als wirksames Antidot bei Insektizide-Vergiftungen gespritzt. Die Indikation von Atropin erfolgt z.B. auch bei Spasmen und Koliken im Magen-Darm-Kanal, in der Gallenblase oder den abführenden Harnwegen, bei Blasentenesmen, bei Hypersekretion des Magens, bei Magen- und Zwölffingerdarmgeschwüren, bei Asthma bronchiale sowie in höheren Dosen und über längere Zeit auch bei Parkinsonismus. - Ganz allgemein kommt eine automatische Injektionsvorrichtung zur Applikation einer Vielzahl von Injektionsstoffen im human- oder veterinärmedizinischen Bereich in Betracht. Auch ist an Einsatzmöglichkeiten auf technischem Gebiet zu denken. So können insbesondere gefährliche oder giftige Stoffe in weiche Materialien eingebracht werden, wobei z.B. die Konservierung von Weichholz od.dgl. Behandlungen erwähnt werden sollen. Injektionsstoffe können Flüssigkeiten, Gase oer auf Druck verflüssigbare Stoffe sein.

Wesentliche an eine automatische Injektionsvorrichtung zu stellende Forderungen bestehen in Sterilität, Haltbarkeit sowie Stabilität auch über längere Lagerzeiträume, in sehr schnellem und zuverlässigem Durchdringen von mit Schmerzrezeptoren ausgestatteten Gewebeteilen mit der Nadel, ggf. auch nach Durchstoßen von Kleidungsstücken, in gewichtsmäßig leichter und kompakter Bauweise sowie in einfacher und funktionssicherer Handhabung.

Bei einer bekannten gattungsgemäßen Injektionsvorrichtung wird eine Nadel in einer Ampulle durch Festlegung eines Nadelfußes an einem Hubstopfen sowie austrittsseitig in einer Nadelführung und einem Gummistopfen gelagert. Eine Druck-Schraubenfeder greift zum Vortreiben des Hubstopfens in die Ampullenhülse ein. Bei einer solchen Konstruktion ist die Führung und Bewegbarkeit der Nadel infolge der abbremsenden Wirkung des relativ lang sich erstreckenden Gummilagers beeinträchtigt, wobei auch ein auf ihre Ausrichtung und Bewegung sich ungünstig auswirkendes Kraftmoment auftreten kann. Eine am Nadelfuß vorgesehene Kanülenöffnung, d.h. eine dort vorhandene Nadelschwächung, kann zur Verformung oder gar zum Bruch der Nadel führen. Zur ausreichenden Kraftbeaufschlagung des Hubstopfens bzw. zum Antrieb der Nadel ist eine verhältnismäßig große Kraft erforderlich, die von der relativ lang sich erstreckenden Druck-Schraubenfeder mit relativ großem Windungsdurchmesser aufgebracht werden muß, wobei die Feder in einer die Ampulle und sie selbst lagernden inneren Hülse dem Stopfen nachgeordnet ist und beim Betrieb in die Ampulle eingreift. Um der Kraftbeanspruchung standzuhalten, besteht der Ampullenkörper aus rostfreiem Stahl. Infolge katalytischer Wirkungen sowie chemischer und/oder galvanischer Reaktionen des Metalls mit dem Injektionsstoff wird dessen Stabilität und Wirksamkeit gesenkt. Für viele Injektionsstoffe ist rostfreier Stahl nicht inert, so daß er insoweit als Ampullenmaterial ausscheidet. Die Druckeinrichtung und damit die Gesamtvorrichtung bauen relativ großräumig, wobei der Kraft-Wirkungsgrad und die Kraftführung durch konstruktive Gegebenheiten eingeschränkt und ungünstig sind. Die Bauweise bringt auch die Gefahr nicht ausreichender Keimfreiheit mit sich und steht einer Wiederverwendung oder Neuherstellung der Vorrichtung entgegen. Bei einer weiteren bekannten Injektionsvorrichtung (DE-B- 14 91 842) umfaßt die Nadellagerung einen Tauchkolben mit einem verformbaren, in einen Zylinderraum zurückweichenden Kolben, so daß Lagerung und Bauweise aufwendig sowie Verwendbarkeit und Leistungsfähigkeit durch Konstruktions- und Materialerfordernisse eingeschränkt sind.

Andere, nicht gattungsgemäße automatische Injektionsvorrichtungen umfassen eine fest mit einer bewegbaren Ampulle verbundene Nadel (DE-A- 24 31 347, DE-B- 19 34 117, DE-C- 24 36 000), wobei insbesondere auf einen Nadelhalter eine Kappe einer Injektionskanüle aufgesteckt ist (DE-A- 33 42 407).

Demgegenüber liegt der Erfindung die Aufgabe zugrunde, eine automatische Injektionsvorrichtung zu schaffen, die einen Hochgeschwindigkeitsaustritt der Injektionsnadel unter gleichmäßiger und definierter Injektionsstofffreigabe gewährleistet, wobei Anordnung und Ausbildung von Nadellagerung Einsatz- und Verwendungsmöglichkeiten bzw. Konstruktion und Beschaffenheit von Nadel, Patrone, Antriebs- bzw. Druckeinrichtung, Auslöseeinrichtung und Sicherheitseinrichtung optimal nutzbar machen oder verbessern sollen und dabei die Gesamtvorrichtung bei langer Lagerfähigkeit sowie bei Keimfreiheit gewährleistender Bauweise mit besonders hohem mechanischem Wirkungsgrad funktionssicher, gewichtsmäßig leicht, robust, kompakt, kleinbauend sowie einfach, schnell und risikolos handhabbar sowie durch die Möglichkeit eines Patronenaustausches und der erneuten Verwendung von Vorrichtungsteilen wiederverwendbar werden soll. Ein weiteres Ziel der Erfindung besteht darin, eine für sich handhabbare kompakte Patrone oder Ampulle zu schaffen, die mühelos als selbständiges Bauteil in die Injektionsvorrichtung einsetzbar sein und die wahlweise Verwendbarkeit der Injektionsvorrichtung für eine Vielzahl von Injektionsstoffen, eröffnen soll, wobei eine einfache und funktionssichere sowie medizinischen Anforderungen in hohem Maß gerecht werdende Gestaltung erreicht werden soll, so daß sich die Ampulle oder Patrone insbesondere für einen Einsatz in automatischen Injektionsvorrichtungen eignet.

Die Aufgabe wird hinsichtlich einer Injektionsvorrichtung sowie bei einer in eine sol che einsetzbaren Patrone oder Ampulle der eingangs genannten Art dadurch gelöst, daß der Nadelkörper an einem Teil seiner Erstreckung als an der Innenwand der Patrone im Gleitsitz geführte Nadellagerung ausgebildet ist.

Bei der durch den Nadelkörper gebildeten integrierten Nadellagerung ist insbesondere erreicht, daß die Nadel bzw. Kanüle eigenständig für sich in der Ampulle praktisch reibungslos bei einer Art Schwimmwirkung in dem Injektionsstoff bewegbar und geführt ist. Dadurch ist die Nadel im wesentlichen verzögerungsfrei sehr schnell antreibbar, und es ist eine hohe Austrittsleistung erzielbar. Die erfindungsgemäße Nadellagerung reicht allein zur Führung und Positionierung der Nadel aus, so daß gesonderte weitere Nadellager, insbesondere im Ampullenaustrittsbereich entfallen. Zudem ist die Nadel beim Betrieb vor Beschädigung oder Zerstörung gesichert, und ihre Bewegung sowie Ausrichtung sind weitgehend von Einflüssen der Lagerung des Hubelements bzw. dessen Bewegungsverhaltens befreit. D. h., daß die Nadellagerung zu dem Hubelement bzw. zu einer Druckeinrichtung mechanisch derart entkoppelt ist, daß die Kraftübertragung an die Nadel ohne nachteilige Auswirkung auf deren Ausrichtung sowie ihre Beschädigung vermeidend erfolgt. Dies führt zu einer besonders einfach und kompaktbauenden, robusten, leistungsfähigen und funktionssicheren und leicht handhabbaren Ampulle und zu einer Vorrichtung mit entsprechenden Vorteilen, deren Einzelteile im Hinblick auf Keimfreiheit sowie Betrieb und auch Wiederverwendbarkeit in besonderem Maß unbeeinträchtigt durch die Nadellagerung, aber in optimaler Anpassung an und Abstimmung auf diese vorgesehen werden können.

Soweit ein, für eine automatische Injektion an sich nicht erforderlicher, in jedem Fall genau zentrischer Austrieb der Nadel gewünscht ist, kann innerhalb der Ampulle, vorzugsweise im Austrittsbereich der Nadel, eine die Nadelausrichtung unterstützende Distanzscheibe angeordnet sein, die die erfindungsgemäß erzielte, im wesentlichen reibungsfreie Bewegbarkeit der Nadel nicht beeinträchtigt.

Eine erfindungsgemäße Ausgestaltung der Nadellagerung besteht darin, daß sie als Hubelement oder antriebsseitiges Abdichtelement ausgebildet ist, wobei sie in einen Hubstopfen integriert oder mit einer dem Nadelvortrieb dienenden, insbesondere mit der Kraft eines Fluids beaufschlagbaren, vorzugsweise dünnwandigen Fläche versehen ist. Damit wird die Ampulle besonders kleinbauend, und die Nadellagerung kann zur exakten Führung und Ausrichtung des Hubstopfens dienen bzw. selbst als Hubelement wirken, wobei der Gleitsitz teilweise die antriebsseitige Abdichtung der Ampulle bildet.

Besonders zweckmäßig ist es, daß die Nadellagerung bei zylinderförmiger Patrone oder Ampulle mindestens eine den Gleitsitz herstellende Kanülenwindung umfaßt, wobei insbesondere eine endseitige Windung an einem als Hubstopfen ausgebildeten Hubelement anliegt. Eine derartige Windungskanüle sorgt in besonderem Maß für eine momentenfreie Eigenlagerung der Nadel, wobei die Gleitfläche und damit auch die Reibungsfläche unmittelbar an der Ampulleninnenwand minimiert sind. Eine einzige Windung führt zu einer besonders kurzen Kanüle bzw. Nadel. Je nach gewünschtem Restverdrängungsraum können zwei oder mehrere Windungen eng aneinanderliegend oder im Abstand zueinander geformt sein. Insbesondere in Serienfertigung ist eine besonders maßgenaue Ausrichtung der Nadelspitze senkrecht zu dem Windungskörper im Hinblick auf einen mittigen Durchtritt durch die Ampullendichtung gewährleistet. Infolge der Ausbildung der Nadellagerung als Kanülen windung kann die Nadel in besonders einfacher Weise auch unter einem Winkel von insbesondere 7-15° gegenüber der Patronen-/Ampullenachse bzw. der Windungsachse ausgerichtet sein. Die Größe der eingestellten Winkelabweichung (Deviation) aus der Achse erfolgt nach Maßgabe der anatomischen Gegebenheiten. Z.B. ist eine relativ große Nadeldeviation für eine Autoinjektion am Gesäß zweckmäßig. Die Anlage der endseitigen Windung an dem Hubstopfen bewirkt eine verzögerungsfreie, d.h. schnelle und unter hoher Kraft mögliche Kraftübertragung. Eine Anordnung der Kanülen-Eintrittsöffnung am stopfenseitigen Windungsende stellt ein Optimum an Flüssigkeitseintritt in und Flüssigkeitsdurchtritt durch die Kanüle sicher.

Eine Injektionsstoffmenge sowie eine gewünschte Applikationstiefe für die Nadel lassen sich zweckmäßig durch die Kanülenwindungszahl und in Abstimmung damit mit einem kompressiblen Gas- oder Totvolumen, d.h. insbesondere auch mit einem den Injektionsstoffaustritt definiert verzögernden Gas- oder Luftkissen, vorgeben.

In Weiterbildung der Erfindung ist vorgesehen, daß die Nadel in einem vorderen separaten, ein Totvolumen bildenden, mit einem Gas oder Gasgemisch gefüllten Ampullenraum liegt, an den sich antriebsseitig, durch ein abdichtendes Hubelement getrennt, ein zwischen diesem und einem Hubstopfen ausgebildeter, den Injektionsstoff enthaltender hinterer Ampullenraum anschließt, und das Hubelement beim Antrieb von einer die Kanülen-Eintrittsöffnung aufweisenden An- oder Ausformung der Nadel und/oder der Nadellagerung durchstoßbar ist. So erhält man eine in einem Luft- bzw. Gasraum trocken und damit steril gelagerte Kanüle. Zudem bewirkt das gegenüber dem Injektionsstoff in größerem Maß kompressible Totvolumen eine sehr kurze definierte Verzögerung des Austrittes des Injektionsstoffes aus der angetriebenen Nadel, d.h., daß die Nadelspitze vor dem Austrieb von Injektionsstoff aus der Ampulle austritt, wobei die Verzögerung durch die Größe des Totvolumens und/oder den Abstand des offenen Nadelendes von dem Hubelement bestimmt ist. Der im vorderen Ampullenteil definiert liegende Gas- oder Totraum gewährleistet eine von der Ausrichtung der Ampulle beim Injizieren unabhängige Verzögerung. Verstopfungsfreiheit der Kanülenöffnung ist insbesondere durch ausreichenden Öffnungsquerschnitt und/oder eine von dem Nadelauftreffbereich abgewandte Öffnung gewährleistet. Die das Nadelende bildende An- oder Ausformung kann zum leichten Durchdringen des Hubelementes mit einem Schliff versehen und/oder spitz ausgeführt sein.

Eine besondere Ausgestaltung des Erfindung besteht darin, daß innerhalb der Ampulle, insbesondere zwischen Nadellagerung und Hubelement ein ein Gasoder Totvolumen bildendes Behältnis wie eine Kapsel mit einer gegenüber Injektionsstoff resistenten, insbesondere durch ihn nicht-lösbaren, durch Auslösen der Hubbewegung leicht zerstörbaren Behältniswand angeordnet ist. Damit ist zunächst ein in der Ampulle stets definiert liegendes gegenüber dem Injektionsstoff ohne Zuhilfenahme von Hub- oder Stopfenmitteln chemisch und mechanisch abgegrenztes Totvolumen erreicht, das erst bei definierter Druck-Zerstörung freigegeben wird. Zur gezielten Zerstörung des Behältnisses und um bei Injektionsbeginn im wesentlichen den in dem Bereich des zerstörten Behältnisses anfallenden Injektionsstoff in die KanülenEintrittsöffnung - ggf. verzögert infolge ihres sofortigen Eintritts in einen Gas-Totvolumenraum - einströmen zu lassen, ist insbesondere vorgesehen, daß die Nadel und/oder Nadellagerung eine mit der Behältniswand in Eingriff bringbare, bei Druckbeaufschlagung zur Zerstörung des Totvolumen-Behältnisses führende An- oder Ausformung, insbesondere ein spitzförmiges auf die Behältniswand gerichtetes, insbesondere an diese angrenzendes Kanülenende aufweist, wobei insbesondere an der An- oder Ausformung verstopfungsfrei, insbesondere mit ausreichend großem Eintrittsquerschnitt und/oder von der Behältniswand abgewandt, die Kanülen-Eintrittsöffnung zum Einströmen des bei Behältniszerstörung anfallenden Injektionsstoffes vorgesehen ist. Besonders zweckmäßig ist es, daß das Behältnis mindestens eine Substanz enthält, die zusammen mit dem an dem unzerstörten Behältnis anliegenden Ampulleninhalt einen zu applizierenden Injektionsstoff erzeugt. Damit ist erreicht, daß Feststoff, Flüssigkeit und/oder Gas in der Kapsel separiert von dem an dieser anliegenden Injektionsstoff, nämlich einem Lösungsmittel bzw. einem flüssigen Injektionsmittel, bereitgehalten sind und beim definiert eintretenden Kapselzerfall eine Mischung von Flüssigkeiten oder ein Lösen von Feststoff und/oder Gas stattfindet, so daß ein resultierender in die Kanülenöffnung einströmender Injektionsstoff erzeugt ist. So ist die Gabe von separierten und damit insbesondere festen, für sich nicht verflüssigbaren, bei Applikation chemisch miteinander reagierenden Injektionsstoffen eröffnet.

Die Nadeleigenlagerung erlaubt in besonders zweckmäßiger Weise, daß die Ampulle als gegenüber Injektionsstoff chemisch resistentes Behältnis aus Glas oder einem ähnlichen Werkstoff ausgebildet ist. Durch die erfindungsgemäße Art der Nadellagerung ist die Ampullenwand von ungünstigen Kräften befreit, so daß Glas als vorteilhaftes, z.B. durch eine Atropinsulfatlösung nicht angreifbares Material verwendbar ist.

Im Hinblick auf eine besonders kompakte Bauweise, einen Austausch einer Ampulle und/oder für eine Vielzahl von Injektionsstoffen praktisch unbegrenzte Haltbarkeit besteht die besonders vorteilhafte Möglichkeit, daß die Patronen- oder Ampullendichtung der Glas-Ampulle als Silikonplättchen mit einem aus Weißblech gebördelten Rand ausgebildet, die Nadel vollständig im Inneren der Ampulle liegend vorgesehen und das Plättchen von der Nadel durchstoßbar ist. Anstelle von Silikon für das Plättchen bzw. Weißblech für den Bördelrand sind entsprechende gegenüber Injektionsstoffen inerte sowie mechanischen und konstruktiven Erfordernissen genügende Materialien, insbesondere gehärtete Kunststoffe einsetzbar.

Eine mit Austrittsschliff versehene Kanülen-Austrittsöffnung begünstigt besonders ein sehr schnelles Durchdringen von Schmerzrezeptoren aufweisenden Gewebeteilen und stellt durch hohe Schneidwirkung eine Trauma-Minimierung sicher. Zudem bewirkt eine mit der Erfindung erreichbare und definierte Flüssigkeitsfreigabe insbesondere bei geringer Injektionslösungsmenge geringe Gewebsdruckverhältnisse.

Um mit Injektionsflüssigkeiten in Berührung kommende Grenzflächen, wie die Plättchen-Dichtung, die Nadel und ein Hubelement inert zu machen, besteht das Dichtungsplättchen aus Silikon oder aus je nach Injektionsstoff geeignetem Kunststoff, die Kanüle aus auch eine sehr gute Gleitwirkung zwischen Ampulleninnenwand und Nadellagerung bewirkendem V2A- oder V4A-Stahl und der Patronen- oder Ampullenstopfen aus Chlor- oder Chlorbutyl-Kautschuk oder aus je nach Injektionsstoff geeignetem Kunststoffmaterial.

Insbesondere zur einfachen Umrüstung und zum bequemen Austausch der diese Möglichkeiten eröffnenden Ampulle mit integrierter Nadellagerung umfaßt eine erfindungsgemäße automatische Vorrichtung in Weiterbildung als Gehäuse ein Mantelgehäuse und eine dieses im Bereich der Ampullendichtung seitlich abschließende, die Patrone oder Ampulle lagernde Verschlußkappe. Dabei ist es besonders zweckmäßig, daß die Verschlußkappe als in das Mantelgehäuse einsteckbarer, mittels Nut/Feder-Mitteln arretierbarer Schnappverschluß ausgebildet ist.

Bei einer Injektionsvorrichtung mit einer Druckeinrichtung, die eine Druck-Schraubenfeder, eine Federhülse sowie einen infolge Feder-Druckkraft das Hubelement antreibenden Stößel oder Kolben umfaßt, besteht gemäß der Erfindung eine besonders geeignete, durch die erfindungsgemäße Nadellagerung und durch die damit erreichte Ausbildung der Ampulle ermöglichte Ausgestaltung der Vorrichtung darin, daß die Feder zwischen einem Mantelgehäuse und der Federhülse angeordnet sowie gespannt gehalten ist, die Federhülse an dem M antelgehäuse verschiebbar gelagert sowie mit dem Stößel wirkungsverbunden ist und die den Nadelkörper im Gleitsitz unmittelbar an ihrer Innenwand führende und lagernde Patrone oder Ampulle derart angeordnet ist, daß sie sich in dem Anordnungsbereich der gespannten Feder hinein erstreckt, wobei sie insbesondere die Ampulle umgreifend, also insoweit direkt darüber vorgesehen ist. Diese Anordnung führt zu einer besonders robusten, funktionstüchtigen, kleinbauenden, eine hohe Feder- bzw. Antriebskraft gewährleistenden und damit leistungsfähigen Injektionsvorrichtung. Insbesondere erreicht man so, daß nur der Stößel in die Patrone oder Ampulle hineingetrieben wird, während die Feder auch bei Entspannung derselben außerhalb der Patrone zwischen Mantelgehäuse und Federhülse gesondert gelagert ist, d. h. außerhalb der Ampulle in Aktion tritt. Dabei sorgt die den Stößel antreibende Federhülse für eine besonders stabilisierte Kraftbeaufschlagung, d.h. einen störungsfreien, genau ausgerichteten geradlinigen Stößelantrieb. Die Kraftwirkung und -übertragung sowie die Raumanordnung innerhalb der Vorrichtung sind besonders günstig, da die Kraftquelle, d.h. die Feder, auch im gespannten Zustand raumsparend in der Nähe des Ampullenhubelements außenliegend zwischen Mantelgehäuse und Ampulle vorgesehen ist. Damit ist die Ampulle sehr stoßsicher und infolgedessen bruchsicher gelagert. So ist eine besonders starke Ausführung bzw. Dimensionierung der Feder möglich, d.h. eine Schraubenfeder mit relativ großem Durchmesser und dickem Federmaterial bei Spannbarkeit auf kleinstem Raum, wobei aber die besonders kleinbauende Bauweise der Gesamtvorrichtung nicht beeinträchtigt ist. Die freie, eigenständige Ampullen-Nadellagerung gewährleistet eine derartige komprimierte und hohe Antriebskraft sicherstellende Bauweise. Diese wird insbesondere dadurch erreicht, daß das Mantelgehäuse als zylinderförmige abgestufte Hülse ausgebildet ist mit einem im wesentlichen die Ampulle umgebenden vorderen Abschnitt und einem an diesen über eine Schulter anschließenden hinteren Abschnitt mit zu dem vorderen Gehäuseabschnitt um die Schulter kleineren Durchmesser, die Federhülse in dem hinteren Gehäuseabschnitt sowie auf der zylinderförmig ausgebildeten Ampulle bewegbar gelagert ist und diese im gespannten Zustand der Druckeinrichtung im wesentlichen entlang der Erstrekkung der gespannten Druck-Schraubenfeder übergreift, wobei die Feder zwischen der Schulter und einem ringförmigen, am übergreifenden Ende der Federhülse angeordneten Ansatz gespannt gehalten ist. Die Bauweise erlaubt, daß der Kolben des Stößels im gespannten Zustand der Vorrichtung vor dem innenseitigen Ende der Ampulle liegend außerhalb derselben angeordnet ist und dabei ein kolbenseitiges Ampullen-Hubelement, insbesondere ein Hubstopfen, im wesentlichen mit diesem Ende abschließt, wobei nadelseitig ein zweites, mit dem kolbenseitigen Hubelement einen kompressiblen Raum einschließendes Hubelement vorgesehen sein kann, und der Stößel an seinem rückwärtigen Ende an der Federhülse, dadurch in Richtung der Nadel treibbar, gelagert ist. Man erkennt, daß die Vorrichtung vorteilhaft mit einer räumlich nach außen abgeschlossenen Ampulle baut, wobei die Kraft an das nadelseitige Hubelement über einen geeigneten kompressiblen Raum übertragen werden kann. Auch ist es möglich, daß der Stößel im gespannten Zustand der Vorrichtung in die Ampulle hineinführend die Schraubenfeder durchgreift, dabei mit seinem Kolben an dem Stopfen anliegt und an seinem anderen rückwärtigen Ende an der Federhülse, dadurch in Richtung der Nadel treibbar, gelagert ist. Das hintere verjüngte Gehäuseteil bildet ein raumsparendes, Bewegung stabilisierendes Hülsen-Gleitlager für die Federhülse aus, während zudem der Platzbedarf für die Druck-Schraubenfeder kleingehalten ist und diese raumsparend und raumnutzend zwischen Gehäusemantel und Ampulle parallel zu dieser liegt, wobei sich ihr Betrieb infolge der Außenanordnung auf der Federhülse nicht nachteilig auswirken kann, so daß insbesondere eine durch die erfindung sgemäße Art der Nadellagerung ermöglichte, an sich zerbrechliche Glasampulle verwendbar ist.

Die erfindungsgemäße Nadellagerung bzw. Ampullenausbildung ermöglichen in Weiterbildung der Erfindung und zwar insbesondere im Hinblick auf eine besonders kompakte sowie Keimfreiheit gewährleistende Bauweise der Vorrichtung und/oder insbesondere hinsichtlich einer Wiederverwendbarkeit von Vorrichtungsteilen, daß in an sich bekannter Weise die Auslöseeinrichtung eine in Achsrichtung verschiebbar auf einem Mantelgehäuse angeordnete Auslösehülse und ein damit bewegbares Auslöseelement swie ein mit der Druckeinrichtung verbundenes, an dem Mantelgehäuse arretiertes, dadurch die Druckeinrichtung in gespanntem Zustand haltendes Federelement umfaßt, wobei unter Anschlag des Auslöseelements gegen das Federelement dieses aus seinem Federsitz lösbar und damit die Druckeinrichtung auslösbar ist. Dabei ermöglicht die erwähnte Unterteilung des Mantelgehäuses in einen vorderen und hinteren Gehäuseabschnitt zur weiteren Verbesserung der handlichen und praktischen Verwendung der Selbstinjektionsspritze, daß die Auslösehülse kleinbauend und einfach bedienbar nur auf dem hinteren abgesetzten Gehäuseabschnitt im Gleitsitz in Richtung der Schulter verschiebbar gelagert ist.

Zur Vermeidung einer ungewollten Selbstauslösung der Spritze kann die erfindungsgemäße Vorrichtung bei besonders zuverlässiger und wirksamer Arbeitsweise mit einer an sich bekannten Sicherheitseinrichtung ausgestattet werden, die ein Sicherungselement mit einem an dem Stößel angreifenden und diesen gegen die Kraft der Feder arretiert haltenden Eingriffselement umfaßt. Dabei ist es hinsichtlich leichter Montagemöglichkeiten und auch unter Berücksichtigung einer Neuverwendung besonders zweckmäßig, daß das Sicherungselement als Sicherungsknopf auf einem an der Auslösehülse stirnseitig ausgebildeten, zugleich das Auslöseelement bildenden Wulstrand aufgesetzt ist und das Eingriffselement als Knopfstift oder -stopfen in das rückwärtige, als das Auslöse-Federelement ausgebildete Ende des Stößels eingreift.

Die Einzelteile der erfindungsgemäßen Vorrichtung sowie diese selbst lassen sich in Serienfertigung kostengünstig herstellen. Die konstruktive Kombination der Teile führt zu einer besonders risikolos arbeitenden, kurzbauenden Vorrichtung, wobei für eine Injektion erforderliche Sterilität bzw. Keimfreiheit erreicht sind. Preiswerte Gehäuseteile aus Kunststoff sind lichtundurchlässig ausführbar, so daß auch bei Verwendung vorteilhafter Glasampullen Lichtschutz und damit Langzeitlagerung gewährleistet sind. Eine Druck-Schraubenfeder ist zweckmäßig als verzinkte Stahlfeder ausgeführt, die insbesondere einen hohen Federdruck aufweisen kann, ohne daß die Betriebssicherheit bzw. Funktionstüchtigkeit der Einzelteile und insbesondere der Ampulle beeinträchtigt sind. Die Glasampullenausführung führt zu einer nur schwach magnetischen, also auch insoweit von außen nicht unerwünscht beeinflußbaren Ampulle bzw. Vorrichtung. Die mit der Erfindung erreichten Vorteile bestehen insbesondere darin, daß ein Injektionsspritzenautomat und eine Patrone oder Ampulle geschaffen sind, die bei Keimfreiheit und hohe Lagerstabilität gewährleistender Bauweise besonders handlich, praktisch, funktionssicher und leistungsfähig sind, wobei die Ampulle definierte Anordnungen von Gas- oder Totvolumen ermöglicht sowie die Verwendung von praktisch allen in Frage kommenden, insbesondere auch von separierten, zu mischenden oder zu lösenden Injektionsstoffen eröffnet. Laien ist die Möglichkeit einer fast schmerzlosen Gabe einer entweder subkutanen oder intramuskulären Eigeninjektion eröffnet. Dabei besteht die Möglichkeit einer einfachen Umrüstung und des Austausches von Injektionsampullen zusammen mit der Wiederbenutzung von Vorrichtungsteilen. Die erfindungsgemäße Nadel/ Ampullengestaltung bzw. die damit erreichbare Materialausführung für eine Ampulle sowie die mit Druckeinrichtung, Auslöseeinrichtung und Sicherheitseinrichtung sich ergebende einfache und zuverlässige Bau- bzw. Funktionsweise ermöglichen für eine Vielzahl von Injektionsstoffen praktisch unbegrenzte Haltbarkeit sowie einen besonders sicheren und den besonderen Erfordernissen der medizinischen Anwendung gerecht werdenden Betrieb, wobei die Kanüle Schmerzfreiheit sicherstellend sehr schnell austritt und je nach Placierung eines Injektors ggf. Kleidung und einige Millimeter Hautfläche durchdringen kann, ohne daß bei Vorsehen eines Totvolumens zunächst Injektionsflüssigkeit austritt, dann aber ein sehr schneller und gezielter Austrieb der Flüssigkeit stattfindet.

Weitere Zweckmäßigkeiten, Ausgestaltungen und Ausführungsbeispiele der Erfindung gehen aus der im folgenden beschriebenen schematischen Zeichnung hervor. Es zeigt

Fig. 1      in Seitenansicht einen Schnitt durch eine erfindungsgemäße Injektionsvorrichtung mit einer darin gelagerten erfindungsgemäßen Ampulle,

Fig. 2 bis 4      Ansichten von erfindungsgemäßen Injektionsnadeln,

Fig. 5a u.5b      eine erfindungsgemäße Ampulle mit steril gelagerter Nadel sowie

Fig. 6a u.6b      eine erfindungsgemäße Ampulle mit Kapsel und schwimmender Nadel.

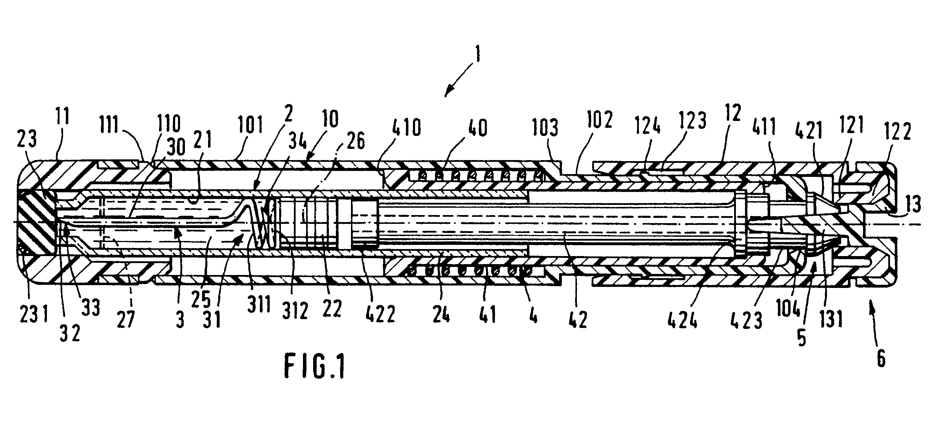

Gemäß Fig. 1 umfaßt eine selbsttätige Injektionsvorrichtung 1, die sich im gespannten Zustand befindet, ein einstückiges zylinderförmiges Mantelgehäuse 10 mit einem vorderen Gehäuseabschnitt 101 und einem zu diesem über eine Schulter 103 abgestuften hinteren Gehäuseabschnitt 102 geringeren Durchmessers. Das vordere Ende des Spritzenautomaten 1 ist mit einer eine erfindungsgemäße Ampulle 2 lagernden Verschlußkappe 11 abgeschlossen, die in den Gehäuseabschnitt 101 hineingesteckt und mit einem Nut/Feder-Mittel 110/111 aufweisenden, entriegelbaren Schnappverschluß arretiert ist. Auf dem hinteren Gehäuseabschnitt 102 ist im Gleitsitz eine endseitig mit einem Sicherungsknopf 13 als Sicherungselement abgeschlossene Auslösehülse 12 vorgesehen, die mit einer Ausnehmung 123 in der Hülse und mit einer umlaufenden Gehäuseverdickung 124 an dem hinteren Gehäuseabschnitt 102 gehalten ist. Dieser lagert in seinem Inneren im Gleitsitz eine zylinderförmige Federhülse 41, die ihrerseits die Ampulle 2 im Gleitsitz lagert und sich über diese hinweg bewegen kann.

Die in Fig. 1 dargestellte erfindungsgemäße Ampulle oder Patrone 2 ist als zylinderförmiges Glasbehältnis ausgeführt, das an der Ampullenvorderseite mit einem einen aus Weißblech gebördelten Rand 231 umfassenden Silikonplättchen 23 nach außen abgedichtet ist und mit einer Injektionsflüssigkeit 25, insbesondere einer Injektionslösung aus Atropinsulfat, Säure zur pH-Wert-Einstellung, z.B. Salzsäure, einem geeigneten Salz zur Isotonisierung, z.B. Natriumchlorid, und Aqua pro Injektione, zwischen der Dichtung 23 und einem die Flüssigkeit abdichtenden Hubstopfen 22 als Hubelement gefüllt ist. Glas als Material für die Ampulle 2 gewährleistet über einen langen Zeitraum die Erhaltung einer sterilen und pyrogenfreien Injektionslösung mit geeigneter Tonizität und Erhaltung des gewünschten pH-Wertes bei Gewährleistung von Stofffreiheit gemäß standardisierten Reinheitskriterien. Infolge geringer Wärmeleitfähigkeit des Glasbehältnisse 2 ist eine Wärmeübertragung an die Injektionsflüssigkeit, die bei herkömmlichen Metallampullen auftreten kann, vermieden, so daß die Flüssigkeit vor Zersetzung durch hohe Temperatur geschützt und damit auch insoweit stabil gehalten.

Bei der Injektionsvorrichtung gemäß Fig. 1 liegt der in der Ampulle 2 gleitbare Hubstopfen 22 im Bereich vor dem inneren Ende 410 der Federhülse 41, und der hintere Endbereich 24 der Ampulle 2 erstreckt sich in die Federhülse 41 bis in den Bereich der Gehäuseschulter 103. Nach außen liegt der Stopfen 22 an einem Kolben 422 eines Stößels 42 an, der mittels einer umlaufenden Wulst 424 am hinteren Ende 411 der Federhülse 41 in Richtung des Hubstopfens 22 bewegbar, diesen in der Ampulle 2 vortreibbar gelagert ist.

Eine Injektionsnadel 3 ist vollständig im Inneren der Ampulle 2, d.h. in der F lüssigkeit 25 zwischen Dichtung 23 und Hubstopfen 22 liegend, vorgesehen. Sie ist als Windungskanüle mit zwei endseitigen Kanülen-Windungen 311, 312 ausgeführt, die als Windungskörper eine Nadellagerung 31 bilden, indem die Außenflächen der Windungen unmittelbar einen Gleitsitz an der Ampulleninnenwand 21 herstellen. Die äußere Windung 312 bildet eine im wesentlichen plane Anlage für den Hubstopfen 22, so daß der Windungskörper geradlinig in Richtung seiner Achse bzw. der Mittelachse der Ampulle 2 mittels des Hubstopfens 22 nach vorne treibbar ist. Hinsichtlich einer besonders kleinen Bauweise und zur Ausbildung eines Lagers für einen Hubstopfen ist es zweckmäßig, den Windungskörper in den Hubstopfen wenigstens teilweise unter Freilassen der Windungsgleitflächen einzubetten. - Die Windung 311 geht in einen langgestreckten Kanülen-Nadelkörper 30 über, der genau ausgerichtet in der Ampullenmittelachse 26 liegt. Zur Unterstützung einer genau zentrischen Ausrichtung und Führung kann ein die schwimmende Lagerung der Nadel und ihre freie Bewegbarkeit nicht beeinträchtigendes Distanzelement, insbesondere eine gestrichelt dargestellte Distanzscheibe 27, im vorderen Bereich der Ampulle vorgesehen sein. Die Kanüle ist am Ende der Windung 312 durch eine im Bereich der Anlage der Nadellagerung 31 an dem Hubstopfen 22 liegende Kanülen-Eintrittsöffnung 34 offen. Die Nadelspitze ist als mit einem Schliff versehene Kanülen-Austrittsöffnung 33 ausgebildet, die beim Austrieb bzw. Antrieb der Nadel 3 die Dichtung 23 in Achsrichtung 26 durchstößt. Unmittelbar nach dem Durchstoß wird die Flüssigkeit 25 durch die mit den Windungen 311, 312 versehene Kanüle 30, 31 ausgetrieben.

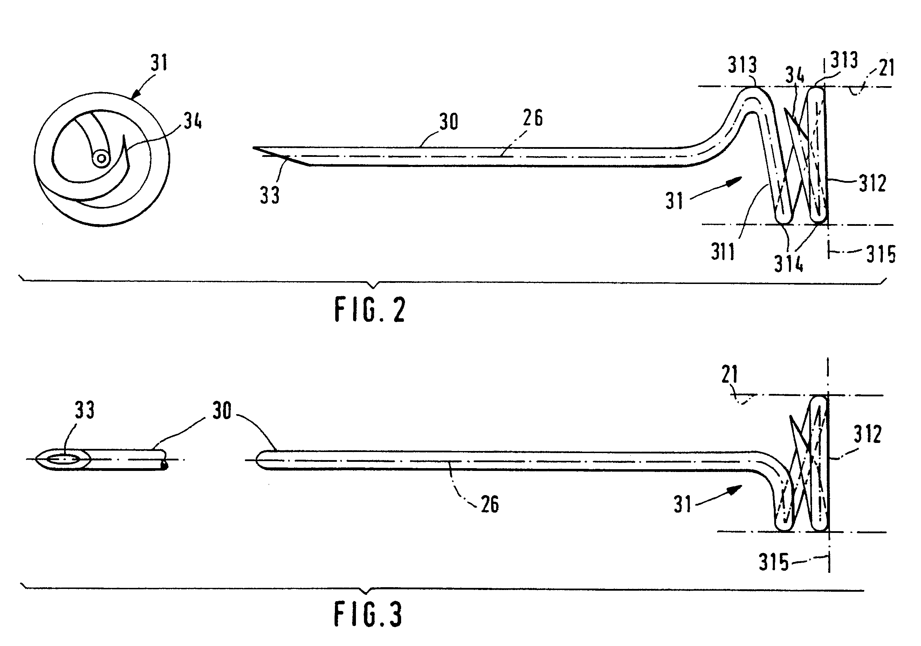

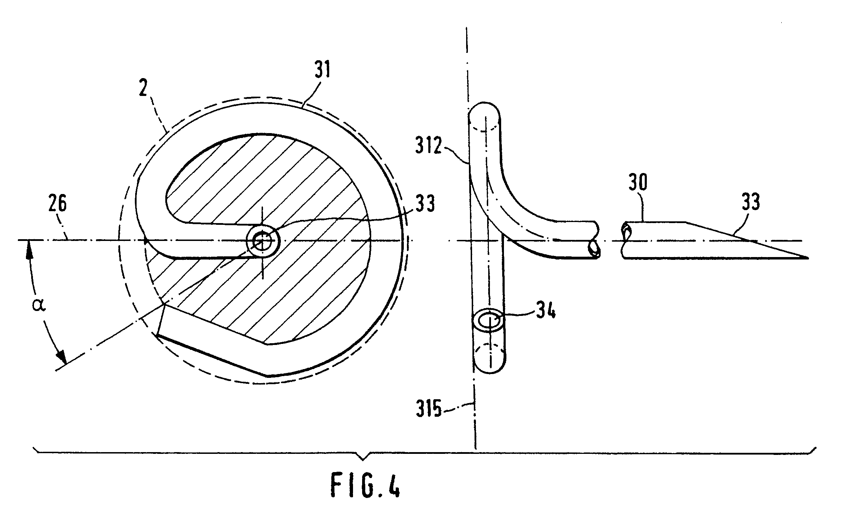

In Fig. 2 ist die in die Ampulle nach Fig. 1 eingesetzte Injektionsnadel 3 in Vorder- und Längsansicht dargestellt. Durch die Nadellagerung 31, d.h. den im wesentlichen zwei Windungen 311 und 312 umfassenden Nadelkörper, sind am Umfang des Windungskörpers in Achsrichtung und quer dazu sich erstreckende, bei 313 und 314 zu erkennende Anlage- oder Gleitflächen für die Ampulleninnenwand 21 ausgebildet. Die Eintrittsöffnung 34 liegt geschützt innerhalb des Windungskörpers 31. Die erfindungsgemäße Nadellagerung kann auch mit einem im wesentlichen nur eine Windung 312 umfassenden Windungskörper 31 ausgeführt sein, wie dies in Fig. 3 dargestellt ist. Ein weiteres Ausbildungsbeispiel für eine Nadel 3 besteht, wie aus Fig. 4 ersichtlich, darin, daß eine endseitige Windung 312 nicht vollständig geschlossen verläuft, indem ein Öffnungswinkel von z.B. 30° verbleibt. Auch bei dieser reduzierten Windungsausbildung ist eine exakte Nadelausrichtung und -führung gewährleistet, da der Nadelkörper bzw. der Nadelschaft 30 insbesondere in einer Injektionsflüssigkeit schwimmt und ebenfalls dadurch zentriert gehalten ist. Die beschriebenen endseitigen Windungen 312 schließen mit endseitig zu der Nadelachse 26 senkrechter Windungsfläche 315 ab. Hinsichtlich einer besonders kurzbauenden Ampulle kann insbesondere auf eine endseitige Windung 312 ein dünnwandiges geschlossenes Flächenelement aufgebracht oder an ihr angeordnet sein, wie dies in Fig. 4 mit einer schraffierten Fläche dargestellt ist. Damit ist die Nadellagerung selbst als Hubelement oder antriebsseitiges Abdichtelement ausgebildet, so daß ein gesonderter Ampullenstopfen entfällt.

In Fig. 1 umfaßte eine Druckeinrichtung 4 der automatischen Injektionsvorrichtung 1 die Federhülse 41, den Stößel 42 sowie eine Druck-Schraubenfeder 40. Diese ist im gespannten Zustand raumsparend außen auf der Federhülse 41 zwischen einem Ringansatz 410 und der Schulter 103 gespannt gehalten und damit zwischen dem Gehäuseabschnitt 101 und der Federhülse 41 im hinteren Endbereich 24 der Ampulle 2 angeordnet. Diese Anordnung und Raumeinteilung erlauben die Verwendung einer stark dimensionierten, d.h. hohe Federenergie aufweisenden Feder. Die Druckeinrichtung 4 bzw. die Feder 40 ist über das rückwärtige Ende 423 des Stößels 42 gespannt, indem es gegen einen endseitigen Öffnungsrand 104 des Gehäuseabschnitts 102 als pilzförmiges Federelement 421 anliegt.

Eine Auslöseeinrichtung 5 umfaßt die Auslösehülse 12 mit einem zu dem Pilzkopf des Federelementes 421 konzentrischem, als Rand 121 ausgebildetem Auslöseelement, der den Pilzkopf durch Anschlag gegen denselben zusammendrückt und damit das Stößel-Federelement 421 bzw. die Stößelbewegung infolge der Kraft der Feder 40 freigibt.

Eine Sicherheitseinrichtung 6 weist als Sicherungselement den in den Rand 121 eingesteckten Sicherungsknopf 13 mit einem Stift oder Stopfen 131 auf, der in eine Nut des Pilzkopf-Federelementes 421 eingreift, so daß es mit dem Auslöseelement 121 nicht zusammendrückbar und damit die Feder in gespanntem Zustand haltend sicher arretiert ist.

Die Gehäuseteile 10, 11, 12 und 13 sowie die Ampullendichtung 23 sind als kostengünstige Kunststoffteile lichtundurchlässig ausgeführt. Die Glaskolben-Ampulle 2 mit der integrierten Gleit-Nadellagerung 31 gewährleistet in Kombination mit der sie lagernden Schnappverschlußkappe 11 sowie der Anordnung und dabei Aufteilung der beschriebenen Bauteile bei Abnehmbarkeit auch der Auslösehülse 12 durch Entriegelung der Sperre 123/124 eine Wieder- oder Neuverwendbarkeit der Vorrichtung 1, indem die kompakte, medizinischen Anforderungen besonders gerecht werdende Ampulle 2 einfach austauschbar und die Feder 40, die als verzinkte Stahlfeder ausgeführt ist, schnell und problemlos wiederspannbar ist, wobei also die Teile 10, 40, 41 und 42 ohne Austausch erneut einsetzbar sind. Die Gesamtbauweise zeichnet sich durch kurze Bauform und geringes Gewicht aus, die die Robustheit des automatischen Injektors nicht beeinträchtigt.

Insbesondere sind mit der erfindungsgemäßen selbsttätigen Injektionsvorrichtung vorteilhaft eine Flüssigkeits-Austrittsmenge von ca. 0,7 g und eine Kanülenaustrittslänge (zwischen Kanülenspitze und Außenwand der Ampullen-Dichtung) von ca. 23 mm erreichbar. Die Kanülenaustrittszeit kann bei einem Federdruck von ca. 10,5 N/m² im Bereich von 0,5 s liegen. Die kompakte Bauweise ermöglicht insbesondere eine Gesamtlänge von ca. 103 mm bei einem Durchmesser von ca. 15 mm. Versuche haben ergeben, daß die Ampulle mit integrierter Windungskanülen-Lagerung gewährleistet, daß bei einem während des Applizierens an der Kanülen-Eintrittsöffnung vorgesehenen Totvolumen die Injektionsflüssigkeit unmittelbar nach Auslösen der Vorrichtung zunächst nicht aus der Kanüle austritt, sondern erst nach Durchdringen von einigen Millimetern Hautfläche ausgetrieben wird. Der Flüssigkeitsaustritt erfolgt also definiert und gleichmäßig und setzt so bei unterschiedlichen Durchblutungs- und Gewebestrukturen ein günstiges physiologisches Depot.

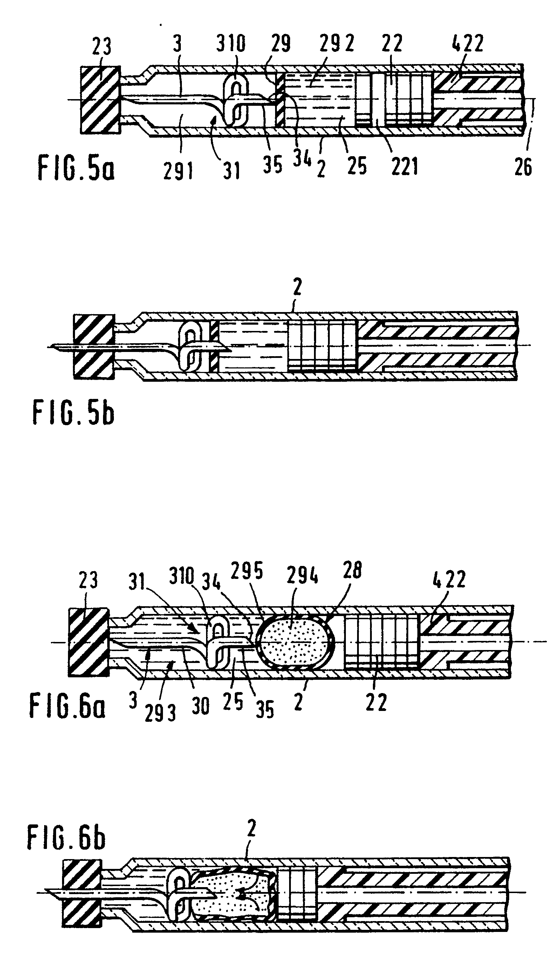

Eine erfindungsgemäße Ampulle gemäß Fig. 5 umfaßt einen Luft-oder Gasraum 291, in dem eine Kanülennadel 3 mit einem eine Windung 310 umfassenden Nadellager 31 steril gehalten, geführt und gelagert ist. An ihn schließt sich über ein Hub-Abdichtelement 29 ein eine Injektionslösung 25 enthaltener Ampullenraum 292 an, der antriebsseitig mit einem Gummistopfen 22 als Hubelement abgeschlossen ist. Wie aus Fig. 5a ersichtlich, umfaßt der Stopfen 22 im unbetätigten Zustand der Ampulle ein Totvolumen oder einen Luftraum 221. Die Nadel 3 weist zur Seite des Abdichtelements 29 eine spießförmige, in der Ampullenachse 26 liegende Ausformung 35 auf, die eine Eintrittsöffnung 34 umfaßt. Infolge der gegenüber dem Injektionslösungsraum 292 leichteren Komprimierbarkeit des Gasraumes 291 wird die Nadel 3 vor Ein-/Durchtritt der Spitze 35 in bzw. durch das HubAbdichtelement 29 auf eine bestimmte Länge flüssigkeitslos durch die Ampullendichtung 23 getrieben und erst dann erfolgt, wie in Fig. 5b gezeigt, ein Einströmen von Flüssigkeit in die Eintrittsöffnung 34 durch die Kanüle hindurch. Die Ausformung 35 bestimmt zusammen mit der Durchtrittsfestigkeit des Hub-Abdichtelements 29 in Abhängigkeit von den Kompressionsgraden und/oder Volumen in den beiden Räumen 291 und 292 den damit definierten verzögerten Flüssigkeitsaustritt.

Eine erfindungsgemäße Kanüle gemäß Fig. 6 umfaßt eine zwischen Injektionsstoff-Raum 293 und Hubstopfen 22 angeordnete Kapsel 28 mit einer chemisch gegen Injektionsflüssigkeit 25 resistenten Kapselwand 295. Infolge Druckbeaufschlagung zerfällt die Kapsel 294 (Fig. 6b), wobei diese Zerstörung durch eine spießförmige An- oder Ausformung 35, die eine Kanülen-Eintrittsöffnung 34 aufweist, eingeleitet wird. Die Kapsel 294 ist wenigstens teilweise mit Feststoff, Flüssigkeit und/oder Gas 294 gefüllt, wobei bei bzw. nach Kapselzerstörung also zwei zuvor separierte Flüssigkeiten gemischt oder ein Feststoff und/oder ein Gas in Flüssigkeit gelöst werden, so daß ein resultierender Injektionsstoff anfällt, der in einer im Kapselzerstörungsbereich sich ausbildenden Konzentration in die Eintrittsöffnung 34 einströmt und beim vollständigen Ausstoß der Nadel 3 ausgetrieben wird. Damit ist die Injektion von getrennt bereitgehaltenen Injektionsstoffen bzw. Injektionsstoffbestandteilen eröffnet, wobei mit einer unmittelbaren Anordnung der An- oder Ausformung 35 bzw. der Eintrittsöffnung 34 an der Behältniswand 295, wie in Fig. 6a dargestellt, ein verzögerter Injektionsstoff-Flüssigkeitsaustritt infolge eines in der Kapsel vorhandenen Gases gewährleistet werden kann. Die erfindungsgemäße Kanülen-Windungslagerung 31 sorgt zusammen mit dem in der Injektionslösung 25 schwimmenden Nadelkörper 30 für eine optimale Lagerung und Führung der Nadel 3.

## Ansprüche

1. Selbsttätige Injektionsvorrichtung (1), mit einer Patrone oder Ampulle (2), die in einem Gehäuse (10) gelagert ist, Injektionsstoff (25) aufnimmt, ein Hubelement (22) aufweist, eine einen langgestreckten Nadelkörper (30) und eine Kanüle (32) umfassende Injektionsnadel (3) umschließt, die durch das Hubelement (22) bewegbar ist und im Bereich ihres antriebsseitigen Endes die Kanülen-Eintrittsöffnung (34) für den Injektionsstoff (25) aufweist, und eine an der Seite der Nadelspitze angeordnete, den Nadelschaft (30) durchlassende Dichtung (23) umfaßt, sowie mit einer an dem Gehäuse (10) gelagerten, das Hubelement (22) mit einer Kraft beaufschlagenden Injektions-Druckeinrichtung (4), mit einer die Druckeinrichtung (4) in Betrieb setzenden Auslöseeinrichtung (5) und mit einer Sicherheitseinrichtung (6) zum Blockieren der Auslöseeinrichtung (5), **dadurch gekennzeichnet, daß** der Nadelkörper (30) an einem Teil seiner Erstreckung als an der Innenwand (21) der Patrone (2) im Gleitsitz geführte Nadellagerung (31) ausgebildet ist.

2. Injektionsvorrichtung nach Anspruch 1, **dadurch gekennzeichnet, daß** innerhalb der Ampulle (2), vorzugsweise im Austrittsbereich der Nadel (3), eine die Nadelausrichtung unterstützende Distanzscheibe (27) angeordnet ist.

3. Injektionsvorrichtung nach Anspruch 1 oder 2, **dadurch gekennzeichnet, daß** die Nadellagerung als Hubelement oder antriebsseitiges Abdichtelement ausgebildet ist, wobei sie in einen Hubstopfen integriert oder mit einer dem Nadelvortrieb dienenden, insbesondere mit der Kraft eines Fluids beaufschlagbaren, vorzugsweise dünnwandigen Fläche versehen ist.

4. Injektionsvorrichtung nach einem der Ansprüche 1 bis 3, **dadurch gekennzeichnet, daß** die Patrone oder Ampulle (2) zylinderförmig ist und die Nadellagerung (31) mindestens eine den Gleitsitz herstellende Kanülenwindung (311, 312) umfaßt.

5. Injektionsvorrichtung nach Anspruch 4, **dadurch gekennzeichnet, daß** die endseitige Windung (312) an einem als Hubstopfen (22) ausgebildeten Hubelement anliegt.

6. Injektionsvorrichtung nach Anspruch 4 oder 5, **dadurch gekennzeichnet, daß** die Kanülenwindungszahl und in Abstimmung damit ein kompressibles Gas- oder Totvolumen so groß vorgesehen sind, daß dadurch die Injektionsstoffmenge sowie eine gewünschte Applikationstiefe für die Nadel vorgegeben sind.

7. Injektionsvorrichtung nach einem der Ansprüche 1 bis 6, **dadurch gekennzeichnet, daß** die Nadel (3) in einem vorderen separaten, ein Totvolumen bildenden, mit einem Gas oder Gasgemisch gefüllten Ampullenraum (291) liegt, an den sich antriebsseitig, durch ein Abdicht-Hubelement (29) getrennt, ein zwischen diesem und einem Hubstopfen (22) ausgebildeten, den Injektionsstoff (25) enthaltender hinterer Ampullenraum (292) anschließt, und das Hubelement beim Antrieb von einer die Kanülen-Eintrittsöffnung (34) aufweisenden An- oder Ausformung (35) der Nadel und/oder der Nadellagerung (31) durchstoßbar ist.

8. Injektionsvorrichtung nach einem der Ansprüche 1 bis 6, **dadurch gekennzeichnet,** daß innerhalb der Ampulle, insbesondere zwischen Nadellagerung (31) und Hubelement (22), ein ein Gas- oder Totvolumen bildendes Behältnis wie eine Kapsel (28) mit einer gegenüber Injektionsstoff (25, 294) resistenten, insbesondere durch ihn nichtlösbaren, durch Auslösen der Hubbewegung leicht zerstörbaren Behältniswand (295) angeordnet ist.

9. Injektionsvorrichtung nach Anspruch 8, **dadurch gekennzeichnet,** daß die Nadel und/oder Nadellagerung eine mit der Behältniswand in Eingriff bringbare, bei Druckbeaufschlagung zur Zerstörung des Totvolumen-Behältnisses führende An- oder Ausformung (35), insbesondere ein spitzförmiges auf die Behältniswand gerichtetes, insbesondere an diese angrenzendes Kanülenende aufweist, wobei insbesondere an der An- oder Ausformung verstopfungsfrei, insbesondere mit ausreichend großem Eintrittsquerschnitt und/oder von der Behältniswand abgewandt, die Kanülen-Eintrittsöffnung zum Einströmen des bei Behältniszerstörung anfallenden Injektionsstoffes vorgesehen ist.

10. Injektionsvorrichtung nach Anspruch 8 oder 9, **dadurch gekennzeichnet,** daß das Behältnis (28) mindestens eine Substanz enthält, die zusammen mit dem an dem unzerstörten Behältnis anliegenden Ampulleninhalt einen zu applizierenden Injektionsstoff erzeugt.

11. Injektionsvorrichtung nach einem der Ansprüche 1 bis 10, **dadurch gekennzeichnet,** daß die Patrone oder Ampulle (2) als gegenüber Injektionsstoff (25) chemisch resistentes Behältnis aus Glas oder einem ähnlichen Werkstoff ausgebildet ist.

12. Injektionsvorrichtung nach einem der Ansprüche 1 bis 11, **dadurch gekennzeichnet,** daß die Patronen- oder Ampullendichtung der Ampulle als Silikonplättchen (23) mit einem aus Weißblech gebördelten Rand (231) ausgebildet, die Nadel (3) vollständig im Inneren der Ampulle (2) liegend vorgesehen und das Plättchen von der Nadel durchstoßbar ist.

13. Injektionsvorrichtung nach einem der Ansprüche 1 bis 12, **dadurch gekennzeichnet,** daß die Injektionsnadel (3) eine Kanülen-Austrittsöffnung (33) mit Austrittsschliff aufweist und aus V2A-, V4A-Stahl, Teflon, gehärtetem Kunststoff od.dgl. besteht.

14. Injektionsvorrichtung nach einem der Ansprüche 1 bis 13, **dadurch gekennzeichnet,** daß das Hubelement bzw. der Patronen- oder Ampullenstopfen (22) wenigstens teilweise aus Chlor- oder Chlorbutyl-Kautschuk, Silkon oder einem anderen gegenüber Injektionsstoff inerten Material, insbesondere einem gehärteten Kunststoff, besteht.

15. Injektionsvorrichtung nach einem der Ansprüche 1 bis 14, **dadurch gekennzeichnet,** daß das Gehäuse ein Mantelgehäuse (10) und eine dieses im Bereich der Ampullendichtung (23) seitlich abschließende, die Patrone oder Ampulle (2) lagernde Verschlußkappe (11) umfaßt.

16. Injektionsvorrichtung nach Anspruch 15, **dadurch gekennzeichnet,** daß die Verschlußkappe als in das Mantelgehäuse (10) einsteckbarer, mittels Nut/Feder-Mitteln (110/111) arretierbarer Schnappverschluß (11) ausgebildet ist.

17. Injektionsvorrichtung nach einem der Ansprüche 1 bis 16 mit einer eine Druck-Schraubenfeder, eine Federhülse sowie einen infolge Feder-Druckkraft das Hubelement antreibenden Stößel oder Kolben umfassenden Druckeinrichtung, **dadurch gekennzeichnet,** daß die Feder (40) zwischen einem Mantelgehäuse (10) und der Federhülse (41) angeordnet sowie gespannt gehalten ist, die Federhülse an dem Mantelgehäuse verschiebbar gelagert sowie mit dem Stößel (42) wirkungsverbunden ist und die den Nadelkörper (30) im Gleitsitz unmittelbar an ihrer Innenwand (21) führende und lagernde Patrone oder Ampulle (2) derart angeordnet ist, daß sie sich in den Anordnungsbereich der gespannten Feder hinein erstreckt.

18. Injektionsvorrichtung nach Anspruch 17, **dadurch gekennzeichnet,** daß das Mantelgehäuse als zylinderförmige abgestufte Hülse (10) ausgebildet ist mit einem im wesentlichen die Ampulle (2) umgebenden vorderen Abschnitt (101) und einem an diesen über eine Schulter (103) anschließenden hinteren Abschnitt (102) mit zu dem vorderen Gehäuseabschnitt um die Schulter kleineren Durchmesser, die Federhülse (41) in dem hinteren Gehäuseabschnitt sowie auf der zylinderförmig ausgebildeten Ampulle bewegbar gelagert ist und diese im gespannten Zustand der Druckeinrichtung (4) im wesentlichen entlang der Erstreckung der gespannten Druck-Schraubenfeder (40) übergreift, wobei die Feder zwischen der Schulter und einem ringförmigen, am übergreifenden Ende der Federhülse angeordneten Ansatz (410) gespannt gehalten ist.

19. Injektionsvorrichtung nach Anspruch 17 oder 18 **dadurch gekennzeichnet,** daß der Kolben des Stößels im gespannten Zustand der Vorrichtung vor dem innenseitigen Ende der Ampulle liegend außerhalb derselben angeordnet ist und dabei ein kolbenseitiges Ampullen-Hubelement, insbesondere ein Hubstopfen, im wesentlichen mit diesem Ende abschließt, wobei nadelseitig ein zweites, mit dem kolbenseitigen Hubelement einen kompressiblen Raum einschließendes Hubelement vorgesehen sein kann, und der Stößel an seinem rückwärtigen Ende an der Federhülse, dadurch in Richtung der Nadel treibbar, gelagert ist.

20. Injektionsvorrichtung nach Anspruch 17 oder 18 **dadurch gekennzeichnet,** daß der Stößel (42) im gespannten Zustand der Vorrichtung in die Ampulle (2) hineinführend die Schraubenfeder (40) durchgreift, dabei mit seinem Kolben (422) an dem Hubelement (22) anliegt und an seinem anderen rückwärtigen Ende (423) an der Federhülse (41), dadurch in Richtung der Nadel (3) treibbar, gelagert ist.

21. Injektionsvorrichtung nach einem der Ansprüche 1 bis 20, **dadurch gekennzeichnet,** daß die Auslöseeinrichtung (5) eine in Achsrichtung verschiebbar auf einem Mantelgehäuse (10) angeordnete Auslösehülse (12) und ein damit bewegbares Auslöseelement (121) sowie ein mit der Druckeinrichtung (4) verbundenes, an dem Mantelgehäuse arretiertes, dadurch die Druckeinrichtung in gespanntem Zustand haltendes Federelement (421) umfaßt, wobei unter Anschlag des Auslöseelements gegen das Federelement dieses aus seinem Federsitz lösbar und damit die Druckeinrichtung auslösbar ist.

22. Injektionsvorrichtung nach einem der Ansprüche 18 bis 21, **dadurch gekennzeichnet,** daß eine Auslösehülse (12) auf dem hinteren abgesetzten Gehäuseabschnitt (102) im Gleitsitz in Richtung der Schulter (103) verschiebbar gelagert ist.

23. Injektionsvorrichtung nach einem der Ansprüche 17 bis 22, **dadurch gekennzeichnet,** daß die Sicherheitseinrichtung (6) ein Sicherungselement (13) mit einem an dem Stößel (42) angreifenden und diesen gegen die Kraft der Feder (40) arretiert haltenden Eingriffselement (131) umfaßt.

24. Injektionsvorrichtung nach Anspruch 23, **da-durch gekennzeichnet,** daß das Sicherungs-element als Sicherungsknopf (13) auf einem an einer Auslösehülse (12) stirnseitig ausgebildeten, zugleich das Auslöseelement (121) bildenden Wulstrand (122) aufgesetzt ist und das Eingriffselement als Knopfstift oder -stopfen (131) in das rückwärtige, als das Auslöse-Federelement (421) ausgebildete Ende (423) des Stößels (42) eingreift.

25. Patrone oder Ampulle (2) für eine Injektionsvorrichtung (1), insbesondere eine selbsttätige Injektionsspritze, wobei die Patrone oder Ampulle (2) Injektionsstoff (25) aufnimmt, ein Hubelement (22) aufweist, eine einen langgestreckten Nadelkörper (30) und eine Kanüle (32) umfassende Injektionsnadel (3) umschließt, die durch das Hubelement (22) bewegbar ist und im Bereich ihres antriebsseitigen Endes die Kanülen-Eintrittsöffnung (34) für den Injektionsstoff (25) aufweist, und eine an der Seite der Nadelspitze angeordnete, den Nadelschaft (30) durchlassende Dichtung (23) umfaßt, **dadurch gekennzeichnet,** daß der Nadelkörper (30) an einem Teil seiner Erstreckung als an der Innenwand (21) der Patrone (2) im Gleitsitz geführte Nadellagerung (31) ausgebildet ist.

26. Patrone oder Ampulle nach Anspruch 25, **dadurch gekennzeichnet,** daß innerhalb der Ampulle (2), vorzugsweise im Austrittsbereich der Nadel (3), eine die Nadelausrichtung unterstützende Distanzscheibe (27) angeordnet ist.

27. Patrone oder Ampulle nach Anspruch 25 oder 26, **dadurch gekennzeichnet,** daß die Nadellagerung als Hubelement oder antriebsseitiges Abdichtelement ausgebildet ist, wobei sie in einen Hubstopfen integriert oder mit einer dem Nadelvortrieb dienenden, insbesondere mit der Kraft eines Fluids beaufschlagbaren, vorzugsweise dünnwandigen Fläche versehen ist.

28. Patrone oder Ampulle nach einem der Ansprüche 25 bis 27, **dadurch gekennzeichnet,** daß die Patrone oder Ampulle (2) zylinderförmig ist und die Nadella gerung (31) mindestens eine den Gleitsitz herstellende Kanülenwindung (311,312) umfaßt.

29. Patrone oder Ampulle nach Anspruch 28, **da-**

durch gekennzeichnet, daß die endseitige Windung (312) an einem als Hubstopfen (22) ausgebildeten Hubelement anliegt.

30. Patrone oder Ampulle nach Anspruch 28 oder 29, **dadurch gekennzeichnet,** daß die Kanülenwindungszahl und in Abstimmung damit ein kompressibles Gas- oder Totvolumen so groß vorgesehen sind, daß dadurch die Injektionsstoffmenge sowie eine gewünschte Applikationstiefe für die Nadel vorgegeben sind.

31. Patrone oder Ampulle nach einem der Ansprüche 25 bis 30, **dadurch gekennzeichnet,** daß die Nadel (3) in einem vorderen separaten, ein Totvolumen bildenden, mit einem Gas oder Gasgemisch gefüllten Ampullenraum (291) liegt, an den sich antriebsseitig, durch ein abdichtendes Hubelement (29) getrennt, ein zwischen diesem und einem Hubstopfen (22) ausgebildeter, den Injektionsstoff (25) enthaltender hinterer Ampullenraum (292) anschließt, und das Hubelement beim Antrieb von einer die Kanülen-Eintrittsöffnung (34) aufweisenden An- oder Ausformung (35) der Nadel und/oder der Nadellagerung (31) durchstoßbar ist.

32. Patrone oder Ampulle nach einem der Ansprüche 25 bis 30, **dadurch gekennzeichnet,** daß innerhalb der Ampulle, insbesondere zwischen Lagerung der Nadel und Hubelement (22), ein ein Gas- oder Totvolumen bildendes Behältnis wie eine Kapsel (28) mit einer gegenüber Injektionsstoff resistenten, insbesondere durch ihn nicht-lösbaren, durch Auslösen der Hubbewegung leicht zerstörbaren Behältniswand angeordnet ist.

33. Patrone oder Ampulle nach Anspruch 32, **dadurch gekennzeichnet,** daß die Nadel und/oder Nadellagerung eine mit der Behältniswand in Eingriff bringbare, bei Druckbeaufschlagung zur Zerstörung des Totvolumen-Behältnisses führende An- oder Ausformung, insbesondere ein spitzförmiges auf die Behältniswand gerichtetes, insbesondere an diese angrenzendes Kanülenende aufweist, wobei insbesondere an der An- und Ausformung verstopfungsfrei, insbesondere mit ausreichend großem Eintrittsquerschnitt und/oder von der Behältniswand abgewandt, die Kanülen-Eintrittsöffnung zum Einströmen des bei Behältniszerstörung anfallenden Injektionsstoffes vorgesehen ist.

34. Patrone oder Ampulle nach Anspruch 32 oder 33, **dadurch gekennzeichnet,** daß das Behältnis (28) mindestens eine Substanz enthält, die zusammen mit dem an dem unzerstörten Behältnis anliegenden Ampulleninhalt einen zu applizierenden Injektionsstoff erzeugt.

35. Patrone oder Ampulle nach einem der Ansprüche 25 bis 34, **dadurch gekennzeichnet,** daß die Patrone oder Ampulle (2) als gegenüber Injektionsstoff (25) chemisch nicht resistentes Behältnis aus Glas oder einem ähnlichen Werkstoff ausgebildet ist.

36. Patrone oder Ampulle nach einem der Ansprüche 25 bis 35, **dadurch gekennzeichnet,** daß die Patronen- oder Ampullendichtung der Ampulle als Silikonplättchen (23) mit einem aus Weißblech gebördelten Rand (231) ausgebildet, die Nadel (3) vollständig im Inneren der Ampulle (2) liegend vorgesehen und das Plättchen von der Nadel durchstoßbar ist.

37. Patrone oder Ampulle nach einem der Ansprüche 25 bis 36, **dadurch gekennzeichnet,** daß die Injektionsnadel (3) eine Kanülen-Austrittsöffnung (33) mit Austrittsschliff aufweist und aus V2A-, V4A-Stahl, Teflon, gehärtetem Kunststoff od.dgl. besteht.

38. Patrone oder Ampulle nach einem der Ansprüche 25 bis 37, **dadurch gekennzeichnet,** daß das Hubelement bzw. der Patronen- oder Ampullenstopfen (22) wenigstens teilweise aus Chlor- oder Chlorbutyl-Kautschuk, Silkon oder einem anderen gegenüber Injektionsstoff inerten Material, insbesondere einem gehärteten Kunststoff, besteht.

**Claims**

1. Automatic injection device (1), including a cartridge or ampoule (2), which is mounted in a casing (10), receives an injection substance (25), has a lifting element (22), surrounds an injection needle (3) comprising an elongated needle body (30) and a cannula (32), the needle (3) being movable by the lifting element (22) and having the cannula inlet opening (34) for the injection substance (25) in the region of its drive-side end, and comprises a seal (23) provided on the side of the needle point and permitting the passage of the needle shaft (30); an injection pressure device (4) mounted on the casing (10) and applying a force to the lifting element (22); a release mechanism (5) putting the pressure device (4) into operation; and a safety device (6) for blocking the release mechanism (5), **characterized in** that the needle body (30), over part of its extension, is designed as a needle bearing (31) guided in a sliding fit on the inner wall (21) of the cartridge (2).

2. Injection device according to claim 1, **characterized in** that a spacer disk (27) supporting the needle alignment is provided within the ampoule (2), preferably in the ejection region of the needle (3).

3. Injection device according to claim 1 or 2, **characterized in** that the needle bearing is constructed as a lifting element or a drive-side sealing element, it being integrated into a lifting plunger or provided with a preferably thin-walled surface serving the needle advance and, in particular, being chargeable with the force of a fluid.

4. Injection device according to one of the claims 1 to 3, **characterized in** that the cartridge or ampoule (2) is cylindrical and the needle bearing (31) comprises at least one cannula winding (311, 312) establishing the sliding fit.

5. Injection device according to claim 4, **characterized in** that the terminal winding (312) engages on a lifting element designed as a lifting plunger (22).

6. Injection device according to claim 4 or 5, **characterized in** that the number of cannula windings and, matched thereto, a compressible gas or dead volume are provided to a size that, as a result, the injection substance quantity, as well as a desired administration depth for the needle are predetermined.

7. Injection device according to one of the claims 1 to 6, **characterized in** that the needle (3) is located in a front, separate ampoule space (291) forming a dead volume and filled with a gas or gaseous mixture and to which is connected, on the drive side, a rear ampoule space (292) containing the injection substance (25), separated by a sealing lifting element (29) and designed between the latter and a lifting plunger (22), and the lifting element, when driven, being pierceable by a formation (35) in or on the needle and/or the needle bearing (31), said formation including the cannula inlet opening (34).

8. Injection device according to one of the claims 1 to 6, **characterized in** that within the ampoule, particularly between the needle bearing (31) and the lifting element (22), there is provided a receptacle, such as a capsule (28), forming a gas or dead volume, with a receptacle wall (295) resistant to, in particular, not soluble by the injection substance (25, 294), which can be destroyed easily by initiating the lifting movement.

9. Injection device according to claim 8, **characterized in** that the needle and/or needle bearing has a formation (35) therein or thereon engageable with the receptacle wall and leading to the destruction of the dead volume receptacle upon pressure application, which formation has, in particular, a pointed cannula end directed onto the receptacle wall and, in particular, arranged adjacent thereto, the cannula inlet opening, for the purpose of the inflow of the injection substance occurring upon destruction the receptacle, being provided, in particular, free of obstruction by jamming, on the formation, especially with an adequately large inlet cross-section and/or remote from the receptacle wall.

10. Injection device according to claim 8 or 9, **characterized in** that the receptacle (28) contains at least one substance which, together with the ampoule contents engaging on the undestroyed receptacle, produces an injection substance to be administered.

11. Injection device according to one of the claims 1 to 10, **characterized in** that the cartridge or ampoule (2) is designed as a receptacle chemically resistant to the injection substance (25) and made from glass or a similar material.

12. Injection device according to one of the claims 1 to 11, **characterized in** that the cartridge or ampoule seal is designed as a silicone plate (23) with a beaded tin plate rim (231), the needle (3) being arranged to lie entirely in the interior of the ampoule (2) and the plate capable of being perforated by the needle.

13. Injection device according to one of the claims 1 to 12, **characterized in** that the injection needle (3) has a cannula outlet opening (33) with an outlet grinding and is made from V2A steel, V4A steel, Teflon, hardened plastic, or the like.

14. Injection device according to one of the claims 1 to 13, **characterized in** that the lifting element or the cartridge or ampoule plunger (22) is at least partly made from chlorinated or chlorobutyl caoutchouc, silicone or some other material, in particular a hardened plastic, which is inert with respect to the injection substance.

15. Injection device according to one of the claims 1 to 14, **characterized in** that the casing comprises a jacket casing (10) and a closure cap (11) laterally closing the casing in the region of the ampoule seal (23) and mounting the ampoule or cartridge (2).

16. Injection device according to claim 15, **characterized in** that the closure or cap is designed as a spring catch (11), insertable into the jacket casing (10) and arrestable by means of groove and tongue means (110/111).

17. Injection device according to one of the claims 1 to 16 with a pressure device comprising a helical compression spring, a spring sleeve, as well as a tappet or piston driving the lifting element as a result of spring pressure, **characterized in** that the spring (40) is arranged and kept tensioned between a jacket casing (10) and the spring sleeve (41), the latter is mounted displaceably on the jacket casing and is operatively connected to the piston (42) and the cartridge or ampoule (2) guiding and mounting the needle body (30) in sliding fit is directly arranged on the inner wall (21) thereof, in such a way that it extends into the arrangement area of the tensioned spring.

18. Injection device according to claim 17, **characterized in** that the jacket casing is designed as a cylindrical, stepped sleeve (10) with a front portion (101) substantially surrounding the ampoule (2) and a rear portion (102) following portion (101) via a shoulder (103) and having a diameter smaller by the shoulder as compared with the front casing portion, the spring sleeve (41) is mounted in the rear casing portion and also on the cylindrical ampoule in a moveable manner and overlaps the latter in the tensioned state of pressure device (4) substantially along the extension of the tensioned helical compression spring (40), the spring being held in tensioned state between the shoulder and an annular projection (410) arranged at the overlapping end of the spring sleeve.

19. Injection device according to claim 17 or 18, **characterized in** that, in the tensioned state of the device, the tappet of the piston is positioned to lie upstream of the inner end of the ampoule and outside the latter and that a tappet-side ampoule lifting element, particularly a lifting plunger terminates substantially with said end, a second lifting element enclosing a compressible space with the tappet-side lifting element, when necessary, being provided on the needle side, and that the piston is mounted at its rear end on the spring sleeve and is consequently drivable in the direction of the needle.

20. Injection device according to claim 17 or 18, **characterized in** that, in the tensioned state of the instrument, the piston (42) enters the ampoule (2) and passes through helical spring (40), engages with its tappet (422) on the lifting element (22) and is mounted at its other, rear end (423) on the spring sleeve (41) and is consequently drivable in the direction of needle (3).

21. Injection device according to one of the claims 1 to 20, **characterized in** that the release mechanism comprises a release sleeve displaceably mounted on a jacket casing (10) in the axial direction and a release element (121) movable therewith, as well as a spring element (421) connected to the pressure device (4), arrested on the jacket casing and thereby keeping the pressure device in the tensioned state, the spring element being releasable from its spring seat when the release element strikes against, and, consequently, the pressure device being releasable.

22. Injection device according to one of the claims 18 to 21, **characterized in** that the release sleeve (12) is displaceably mounted on the rear, offset casing portion (102) in sliding fit in the direction of the shoulder (103).

23. Injection device according to one of the claims 17 to 22, **characterized in** that the safety device (6) comprises a safety element (13) with an engagement element (131) engaging on the piston (42) and keeping same locked against the tension of spring (40).

24. Injection device according to claim 23, **characterized in** that the safety element, designed as a safety button (13), is mounted on a bead rim (122) frontally designed on the release sleeve (12) and simultaneously forming the release element (121) and the engagement element, designed as a button pin or plug (131), engages in the rear end (423) of the piston (42), which end is designed as the release spring element (421).

25. Cartridge or ampoule (2) for an injection device, particularly an automatic hypodermic syringe, the cartridge or ampoule (2) receiving injection substance (25), having a lifting element (22), surrounding a hypodermic needle (3) comprising an elongated needle body (30) and a cannula (32), which needle (3) is movable by the lifting element (22) and, in the region of its drive-side end, has a cannula inlet opening (34) for the injection substance (25), and comprising a seal (23) located on the side of the needle point and permitting the passage of the needle shaft (30), **characterized in** that the needle body (30), over part of its extension, is designed as a needle bearing (31) guided in a sliding fit on the inner wall (21) of the cartridge (2).

26. Cartridge or ampoule according to claim 25, **characterized in** that a spacer disk (27) supporting the needle alignment is provided within the ampoule (2), preferably in the ejection region of the needle (3).

27. Cartridge or ampoule according to claim 25 or 26, **characterized in** that the needle bearing is constructed as a lifting element or a drive-side sealing element, it being integrated into a lifting plunger or provided with a preferably thin-walled surface serving the needle advance and, in particular, being chargeable with the force of a fluid.

28. Cartridge or ampoule according to one of the claims 25 to 27, **characterized in** that the cartridge or ampoule (2) is cylindrical and the needle bearing (31) comprises at least one cannula winding (311, 312) establishing the sliding fit.

29. Cartridge or ampoule according to claim 28, **characterized in** that the terminal winding (312) engages on a lifting element constructed as a lifting plunger (22).

30. Cartridge or ampoule according to claims 28 or 29, **characterized in** that the number of cannula windings and, matched thereto, a compressible gas or dead volume are provided to a size that, as a result, the injection substance quantity as well as a desired administration depth for the needle are predetermined.

31. Cartridge or ampoule according to one of the claims 25 to 30, **characterized in** that the needle (3) is located in a front, separate ampoule space (291) forming a dead volume and filled with a gas or gaseous mixture and to which is connected, on the drive side, a rear ampoule space (292) containing the injection substance (25), separated by a sealing lifting element (29), and designed between the latter and a lifting plunger (22) and, when driven, the lifting element being pierceable by a formation (35) in or on the needle and/or needle bearing (31), said formation including the cannula inlet opening (34).

32. Cartridge or ampoule according to one of the claims 25 to 30, **characterized in** that, within the ampoule, particularly, between the needle bearing (31) and the lifting element (22), there is provided a receptacle, such as a capsule (28), forming a gas or dead volume, with a receptacle wall resistant to, in particular, not soluble by the injection substance, which receptacle, can be destroyed easily by initiating the lifting movement.

33. Cartridge or ampoule according to claim 32, **characterized in** that the needle and/or needle bearing has a formation therein or thereon engageable with the receptacle wall and leading to the destruction of the dead volume receptacle upon pressure application, which formation has, in particular, a pointed cannula end directed towards the receptacle wall and, especially, arranged adjacent thereto, the cannula inlet opening for the purpose of the inflow of the injection substance occurring upon destruction of the receptacle, being provided, in particular free of obstruction by jamming, on the formation, particularly with an adequately large inlet cross-section and/or remote from the receptacle wall.

34. Cartridge or ampoule according to claims 32 or 33, **characterized in** that the receptacle (28) contains at least one substance which, together with the ampoule contents engaging on the undestroyed receptacle, produces an injection substance to be administered.

35. Cartridge or ampoule according to one of the claims 25 to 34, **characterized in** that the cartridge or ampoule (2) is designed as a receptacle resistant chemically to the injection substance (25) and made from glass or a similar material.

36. Cartridge or ampoule according to one of the claims 25 to 35, **characterized in** that the cartridge or ampoule seal is designed as a silicone plate (23) with a beaded tin plate rim (231), the needle (3) being arranged to lie entirely within the ampoule (2) and the plate capable of being perforated by the needle.

37. Cartridge or ampoule according to one of the claims 25 to 36, **characterized in** that the injection needle (3) has a cannula outlet opening (33) with an outlet grinding and is made from V2A steel, V4A steel, Teflon, hardened plastic, or the like.

38. Cartridge or ampoule according to one of the claims 25 to 37, **characterized in** that the lifting element or the cartridge or ampoule plunger (22) is at least partly made from chlorinated or chlorobutyl caoutchuc, silicone or some other material, in particular, a hardened plastic, which is inert with respect to the injection substance.

## Revendications

1. Dispositif automatique d'injection (1), comportant une cartouche ou une ampoule (2 ), laquelle est logée dans une enveloppe (10), reçoit une substance injectable (25), comporte un élément de poussée (22), englobe une aiguille pour injection (3), qui comporte un corps d'aiguille de grande longueur (30) et une canule (32), aiguille mobile à travers l'élément de poussée (22) et qui, dans la zone de son extrémité côté actionnement, comporte l'orifice d'entrée de canule (34) destiné à la substance injectable (25), et comporte une étanchéité (23), disposée sur le côté de la pointe de l'aiguille et traversant la tige d'aiguille (30), ainsi qu'un dispositif de compression d'injection (4), logé contre l'enveloppe (10) agissant avec une force sur l'élément de poussée (22), comportant aussi un dispositif de déclenchement qui met en service le dispositif de compression (4), et un dispositif de sécurité (6) destiné à bloquer le dispositif de déclenchement (5), caractérisé en ce que le corps d'aiguille (30) est, sur une partie de son étendue, constitué d'un support d'aiguille (31), guidé en ajustement glissant contre la paroi intérieure (21) de la cartouche (2).

2. Dispositif d'injection selon le revendication 1, caractérisé an ce qu'un disque d'écartement (27), qui aide à l'orientation de l'aiguille, est disposé à l'intérieur de l'ampoule (2), de préférence dans la zone de sortie de l'aiguille (3).

3. Dispositif d'injection selon la revendication 1 ou 2, caractérisé en ce que le support d'aiguille est conçu comme un élément de poussée ou un élément d'étanchéité côté actionnement, ce logement étant intégré dans un bouchon de poussée ou étant pourvu d'une surface servant à l'avance de l'aiguille, pouvant en particulier recevoir la force d'un fluide, et de préférence à faible épaisseur de paroi.

4. Dispositif d'injection selon l'une des revendications 1 à 3, caractérisé en ce que la cartouche ou l'ampoule (2) est cylindrique, et que le support d'aiguille (31) comporte au moins une spire de canule (311, 312) réalisant l'ajustement glissant.

5. Dispositif d'injection selon la revendication 4, caractérisé en ce que la spire en bout (312) s'appuie contre un élément de poussée constitué d'un bouchon de poussée (22).

6. Dispositif d'injection selon la revendication 4 ou 5, caractérisé en ce qu'on prévoit un nombre de spires de canule et, adapté à ce dernier, un volume de gaz ou un volume mort compressible, tels qu'ils prédéfinissent la quantité de substance injectable ainsi qu'une profondeur souhaitée d'application de l'aiguille.

7. Dispositif d'injection selon l'une des revendications 1 à 6, caractérisé en ce que l'aiguille (3) se trouve dans un espace d'ampoule (291), distinct, formant un volume mort, rempli d'un gaz ou d'un mélange de gaz, espace d'ampoule qui se poursuit par un espace d'ampoule arrière (292), côté actionnement, séparé par un élément de poussée d'étanchéité (29) réalisé entre cet élément de poussée et un bouchon de poussée (22), et contenant la substance injectable (25), et que l'élément de poussée peut être transpercé lors de l'actionnement d'un élément rapporté ou d'une déformation (35), présentant l'orifice d'entrée de canule (34), de l'aiguille et/ou du support d'aiguille (31).

8. Dispositif d'injection selon l'une des revendications 1 à 6, caractérisé en ce que, à l'intérieur de l'ampoule, en particulier entre le support d'aiguille (31) et l'élément de poussée (22), est disposé un récipient formant un volume de gaz ou un volume mort, comme par exemple une capsuler (28), présentant une paroi de récipient (295) résistant à la substance injectable (25, 294), en particulier non soluble dans cette dernière et pouvant être facilement détruite par déclenchement du mouvement de poussée.

9. Dispositif d'injection selon la revendication 8, caractérisé en ce que l'aiguille et/ou le support d'aiguille comportent un élément rapporté ou une déformation (35), pouvant entrer en prise avec la paroi du récipient et conduisant, sous l'effet d'une pression, à la destruction du récipient à volume mort, et an particulier une extrémité de canula en forme de pointe, dirigée sur la parci du récipient et notamment délimitant cette dernière, où, en particulier au niveau de l'élément rapporté ou de la déformation, on prévoit, sans colmatage, en particulier avec une section d'entrée suffisamment importante et/ou en un point opposé à la paroi du récipient, l'orifice d'entrée de canule destiné à l'écoulement de la substance injectable qui apparaît lors de la destruction du récipient.

10. Dispositif d'injection selon la revendication 3 ou 9, caractérisé en ce que le récipient (28) contient au moins une substance, qui, avec le contenu de l'ampoule s'appuyant contre le récipient non détruit, forme un produit injectable destiné à être administré.

11. Dispositif d'injection selon l'une des revendications 1 à 10, caractérisé en ce que la cartouche ou l'ampoule (2) est conçue comme un récipient en verre ou en un matériau analogue, chimiquement résistant à la substance injectable (25).

12. Dispositif d'injection selon l'une des revendications 1 à 11, caractérisé en ce que l'étanchéité de la cartouche ou l'ampoule est conçue comme une plaquette de silicone (23) comportant un bord replié en fer blanc (231), que l'aiguille est prévue de façon qu'elle se trouve complètement dans le volume intérieur de l'ampoule (2), et que la plaquette peut être transpercée par l'aiguille.

13. Dispositif d'injection selon l'une des revendications 1 à 12, caractérisé en ce que l'aiguille d'injection (3) comporte un orifice de sortie de canule (33) avec rodage de sortie et est en un acier V2A ou V4A, en Teflon, en une matière plastique durcie ou analogue.

14. Dispositif d'injection selon l'une des revendications 1 à 13, caractérisé en ce que l'élément de poussée ou le bouchon de cartouche ou d'ampoule (22) est, au moins partiellement, en caoutchouc chloré ou chlorobutyle, en silicone ou en un autre matériau inerte vis-à-vis de la substance injectable, notamment en une matière plastique durcie.

15. Dispositif d'injection selon l'une des revendications 1 à 14, caractérisé en ce que l'enveloppe comporte une enveloppe latérale (10) et un couvercle de fermeture (11), qui obture latéralement cette dernière dans la zone de l'étanchéité d'ampoule (23) et qui loge la cartouche ou l'ampoule (2).

16. Dispositif d'injection selon la revendication 15, caractérisé en ce que le couvercle de fermeture est conçu comme un couvercle à fermeture rapide (11), pouvant être inséré dans l'enveloppe latérale (10) et pouvant être bloqué en position par des moyens à languette/ ressort (110/111).

17. Dispositif d'injection selon l'une des revendications 1 à 16, comportant un ressort spirale de compression, une douille élastique et un dispositif de compression comportant un coulisseau ou un piston qui actionne l'élément de poussée en conséquence de la force de compression du ressort, caractérisé en ce que le ressort (40) est disposé et maintenu serré entre une enveloppe latérale (10) et la douille élastique (41), que la douille élastique est logée de façon à pouvoir se déplacer sur l'enveloppe latérale et est reliée au coulisseau (42), et que la cartouche ou l'ampoule (2), qui loge et guide le corps d'aiguille (30) en ajustement glissant directement contre sa paroi intérieure (21), est disposée de façon qu'elle s'étend à l'intérieur de la zone dans laquelle se trouve le ressort tendu.

18. Dispositif d'injection selon la revendication 17, caractérisé en ce que l'enveloppe latérale est conçue comme une enveloppe cylindrique (10) étagée, avec un tronçon avant (101), qui essentiellement entoure l'ampoule (2), et un tronçon arrière (102), qui suit le premier tronçon par l'intermédiaire d'un épaulement (103), le tronçon arrière ayant un diamètre plus petit que celui du tronçon avant, la différence correspondant à l'épaulement, et que la douille élastique (41) est logée d'une manière mobile dans le tronçon arrière et sur l'ampoule de forme cylindrique et recouvre cette dernière, quand le dispositif de compression (4) est tendu, essentiellement le long de l'étendue du ressort spirale de compression tendu (40), auquel cas le ressort est maintenu tendu entre l'épaulement et une saillie annulaire (410), disposée au niveau de l'extrémité en recouvrement de la douille élastique.

19. Dispositif d'injection selon la revendication 17 ou 13, caractérisé en ce que le piston du coulisseau est, quand le dispositif est tendu, disposé en avant de l'extrémité intérieure de l'ampoule et à l'extérieur de cette dernière, et obture ainsi un élément de poussée d'ampoule côté piston, en particulier un bouchon de poussée, essentiellement avec cette extrémité, auquel cas on peut prévoir, côté aiguille, un deuxième élément de poussée, formant avec l'élément de poussée côté piston un espace compressible, et le coulisseau est logé au niveau de son extrémité arrière contre la douille élastique, et donc peut être poussé dans la direction de l'aiguille.

20. Dispositif d'injection selon la revendication 17 ou 18, caractérisé en ce que le coulisseau (42), quand le dispositif est tendu, et passant dans l'ampoule (2), pénètre dans le ressort spirale (40), et de ce fait, par son piston (422), s'appuie contre l'élément de poussée (22) et,au niveau de son autre extrémité arrière (423), est logé contre la douille élastique (41), en pouvant ainsi être poussé dans la direction de l'aiguille (3).

21. Dispositif d'injection selon l'une des revendications 1 à 20, caractérisé en ce que le dispositif de déclenchement (5) comporte une douille de déclenchement (12), disposée, de façon à pouvoir coulisser dans le sens axial, sur une enveloppe latérale (10), et un élément de déclenchement (121) pouvant se déplacer avec cette douille, ainsi qu'un élément élastique (421), relié au dispositif de compression (4), bloqué au niveau de l'enveloppe latérale et de ce fait maintenant le dispositif de compression à l'état tendu, auquel cas, sous l'effet d'une butée de l'élément de déclenchement contre l'élément élastique, ce dernier peut être détaché de son siège de ressort, le dispositif de compression pouvant alors être déclenché.

22. Dispositif d'injection selon l'une des revendications 18 à 21, caractérisé en ce qu'une douille de déclenchement (12) est logée de façon à pouvoir coulisser sur le tronçon d'enveloppe arrière (122), en ajustement glissant dans la directement de l'épaulement (103).

23. Dispositif d'injection selon l'une des revendications 17 à 22, caractérisé en ce que le dispositif de sécurité (6) comporte un élément de sécurité (13) avec un élément d'engrènement (131), en prise avec le coulisseau (42) et maintenant ce dernier bloqué contre la force du ressort (40).

24. Dispositif d'injection selon la revendication 23, caractérisé en ce que l'élément de sécurité, constitué d'un bouton de sécurité (13), est disposé sur un renflement (122), formé côté frontal sur une douille de déclenchement (12) et formant simultanément l'élément de déclenchement (121), et que l'élément d'engrènement, sous la forme d'une cheville ou d'un bouchon-bouton (131), entre en prise avec l'extrémité arrière (423), constituant l'élément élastique de déclenchement (421), du coulisseau (42).

25. Cartouche ou ampoule (2) destinée à un dispositif d'injection 1, en particulier à une seringue pour injection automatique, où la cartouche ou l'ampoule (2) reçoit une substance injectable (25), présente un élément de poussée (22), englobe une aiguille pour injection (3), laquelle comporte un corps d'aiguille longitudinal (30) et une canule (32), aiguille qui peut être déplacée par l'élément de poussée (22) et comporte, dans la zone de son extrémité côté actionnement, l'orifice d'entrée de canule (34) destiné à la substance injectable (25), et comporte une étanchéité (23), disposée sur le côté de la pointe de l'aiguille et laissant passer la tige d'aiguille (30), caractérisée en ce que le corps d'aiguille (30) est, sur une partie de son étendue, conçu comme un support d'aiguille (30), guidé en ajustement glissant contre la paroi intérieure (21) de la cartouche (2).

26. Cartouche ou ampoule selon la revendication 25, caractérisée en ce que, à l'intérieur de l'ampoule (2), de préférence dans la zone de sortie de l'aiguille (3), est disposé un disque d'écartement (27) qui aide à l'orientation de l'aiguille.

27. Cartouche ou ampoule selon la revendication 25 ou 26, caractérisée en ce que le support d'aiguille est conçu comme un élément de poussée ou un élément d'étanchéité côté actionnement, où il est intégré dans un bouchon de poussée ou est pourvu d'une surface servant à la poussée de l'aiguille, pouvant recevoir la force d'un fluide et de préférence à faible épaisseur de paroi.

28. Cartouche ou ampoule selon l'une des revendications 25 à 27, caractérisée en ce que ta cartouche ou l'ampoule (2) a une forme cylindrique, et que le support d'aiguille (31) comporte au moins une spire de canule (311, 312) qui réalise un ajustement glissant.

29. Cartouche ou ampoule selon la revendication 28, caractérisée en ce que la spire en bout (312) s'appuie contre un élément de poussée conçue comme un bouchon de poussée (22).

30. Cartouche ou ampoule selon la revendication 28 ou 29, caractérisée en ce que l'on prévoit le nombre de spires de la canule et, en conséquence, un volume de gaz ou un volume mort compressible, suffisamment grands pour que la quantité de substance injectable, de même que la profondeur d'application souhaitée de l'aiguille, en soient prédéfinies.

31. Cartouche ou ampoule selon l'une des revendications 25 à 30, caractérisée en ce que l'aiguille (3) se trouve dans un espace d'ampoule avant et distinct (291), formant un volume mort, rempli d'un gaz ou d'un mélange gazeux, qui se poursuit côté actionnement, et séparé par un élément de poussée (29) à effet d'étanchéité, par un espace d'ampoule arrière (292), contenant la substance injectable (25) et formé entre l'élément de poussée et un bouchon de poussée (22), et que l'élément de poussée, lors de l'actionnement, peut être traversé par un élément rapporté ou une déformation (35) de l'aiguille et/ ou du support d'aiguille (31), présentant l'orifice d'entrée de canule (34).

32. Cartouche ou ampoule selon l'une des revendications 25 à 30, caractérisée en ce que, à l'intérieur de l'ampoule, en particulier entre le support d'aiguille et l'élément de poussée (22), est disposé un récipient formant un volume de gaz ou un volume mort, de même qu'une capsule (28), comportant une paroi de récipient résistant à la substance injectable, en particulier non soluble dans cette dernière, et pouvant être facilement détruite par le déclenchement du mouvement de poussée.

33. Cartouche ou ampoule selon la revendication 32, caractérisée en ce que l'aiguille et/ou le support d'aiguilla comporte un élément rapporté ou une déformation, en particulier une extrémité de canule pointue, dirigée vers la parci du récipient, et en particulier délimitant cette dernière, pouvant entrer en prise avec la paroi du récipient et conduisant, sous l'effet d'une pression, à la destruction du récipient à volume mort, auquel cas on prévoit, en particulier au niveau de l'élément rapporté et de la déformation, sans colmatage, notamment avec une section d'entrée suffisamment importante et/ou en un point opposé à la paroi du récipient, l'orifice d'entrée de canule destiné à permettre l'écoulement de la substance injectable qui apparaît lors de la destruction du récipient.

34. Cartouche ou ampoule selon la revendication 32 ou 33, caractérisée en ce que le récipient (28) contient au moins un produit qui, avec le contenu de l'ampoule, en contact avec le récipient non détruit, donne une substance injectable destinée à être administrée.

35. Cartouche ou ampoule selon l'une des revendications 25 à 34, caractérisée en ce que la cartouche ou l'ampoule (2) est conçue comme un récipient, qui ne résiste chimiquement pas à la substance injectable (25), en verre ou en un matériau analogue.

36. Cartouche ou ampoule selon l'une des revendications 25 à 35, caractérisée en ce que l'étanchéité de la cartouche ou de l'ampoule est conçue comme une plaquette de silicone (23) avec un bord replié (231) en fer blanc, l'aiguille (3) étant prévue complètement à l'intérieur de l'ampoule (2), et la plaquette pouvant être transpercée par l'aiguille.

37. Cartouche ou ampoule selon l'une des revendications 25 à 36, caractérisée en ce que l'aiguille pour injection (3) comporte un orifice de sortie de canule (33) avec rodage de sortie et est en acier V2A ou V4A, en Teflon, en matière plastique durcie ou analogue.

38. Cartouche ou ampoule selon l'une des revendications 25 à 37, caractérisée en ce que l'élément de poussée ou le bouchon de cartouche ou d'ampoule (22) est, au moins en partie, en caoutchouc chloré ou chlorobutyle, en silicone ou en un autre matériau inerte vis-à-vis de la substance injectable, en particulier une matière plastique durcie.

FIG.1

FIG. 2

FIG.3

EP 0 261 318 B1

FIG.4

EP 0 261 318 B1

FIG.5a

23  3  310  29  292  22  422

291  31  35  34 2  25  221  26

FIG.5b

2

FIG.6a

23  31  310 34  295  294  28  422

3  293  30  25  35  2  22

FIG.6b

2